(19) Europäisches Patentamt
European Patent Office
Office européen des brevets

(11) Veröffentlichungsnummer: **0 012 157**
**A1**

(12) # EUROPÄISCHE PATENTANMELDUNG

(21) Anmeldenummer: **79103165.1**

(22) Anmeldetag: **27.08.79**

(51) Int. Cl.³: **C 07 C 43/215**, C 07 C 43/225, C 07 C 43/243, C 07 C 43/247, C 07 C 41/16, C 07 C 79/355, C 07 C 76/02, C 07 D 317/64, A 01 N 39/00, A 01 N 43/30 // C07C43/23, C07C41/03

(30) Priorität: **28.08.78 CH 9080/78**
**06.04.79 CH 3295/79**
**26.07.79 CH 6922/79**

(43) Veröffentlichungstag der Anmeldung: **25.06.80**
**Patentblatt 80/13**

(84) Benannte Vertragsstaaten: **AT BE CH DE FR GB IT NL**

(71) Anmelder: **CIBA-GEIGY AG, Patentabteilung Postfach, CH-4002 Basel (CH)**

(72) Erfinder: **Brechbühler, Hans U., Dr.,**
**Paracelsusstrasse 63, CH-4058 Basel (CH)**
Erfinder: **Ehrenfreund, Josef, Dr., Langenhagweg 11, CH-4123 Allschwil (CH)**

(74) Vertreter: **Zumstein sen., Fritz, Dr. et al, Bräuhausstrasse 4, D-8000 München 2 (DE)**

(54) **1-Phenoxy-2-alkinyloxy-äthanverbindungen, Verfahren zu ihrer Herstellung, diese Verbindungen enthaltende Mittel und ihre Verwendung zur Bekämpfung von Schädlingen.**

(57) Neue Phenyl-Alkinyläther der Formel

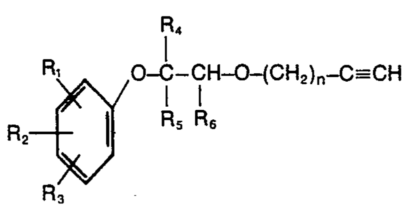

worin

$R_1$ Wasserstoff, $C_1$–$C_4$-Alkyl, Halogen, $C_1$–$C_4$-Alkoxy, $C_3$–$C_4$-Alkenyloxy, $C_3$–$C_5$-Alkinyloxy, Trifluormethyl, Nitro oder Cyano;

$R_2$ Wasserstoff, $C_1$–$C_4$-Alkyl, Halogen, $C_1$–$C_4$-Alkoxy, Trifluormethyl, Nitro oder zusammen mit $R_1$ Methylendioxy;

$R_3$ Wasserstoff, $C_1$–$C_4$-Alkyl oder Halogen;

$R_4$ Wasserstoff oder $C_1$–$C_4$-Alkyl;

$R_5$ Wasserstoff oder $C_1$–$C_4$-Alkyl;

$R_6$ Wasserstoff, $C_1$–$C_4$-Alkyl oder zusammen mit $R_5$ die $-(-CH_2-)_3-$ oder $-(-CH_2-)_4-$Gruppe und

n eine der Zahlen 1, 2 oder 3 bedeuten; Verfahren zur Herstellung dieser Verbindungen sowie diese enthaltende Mittel zur Verwendung in der Schädlingsbekämpfung, insbesondere zur Bekämpfung von Pflanzen und Tiere befallenden Insekten. Die neuen Verbindungen besitzen ovizide und ovolarvizide Wirkung; sie zeigen ferner synergistische Wirksamkeit mit bekannten Insektiziden.

EP 0 012 157 A1

BEZEICHNUNG GEÄNDERT
siehe Titelseite

CIBA-Geigy AG — 5-11997/1-3

Basel (Schweiz)

## Phenyl-Alkyläther

Die vorliegende Erfindung betrifft neue 1-Phenoxy-2-alkinyloxy-äthane, Verfahren zu ihrer Herstellung und ihre Verwendung in der Schädlingsbekämpfung.

Die erfindungsgemässen 1-Phenoxy-2-alkinyloxy-äthane haben die Formel I

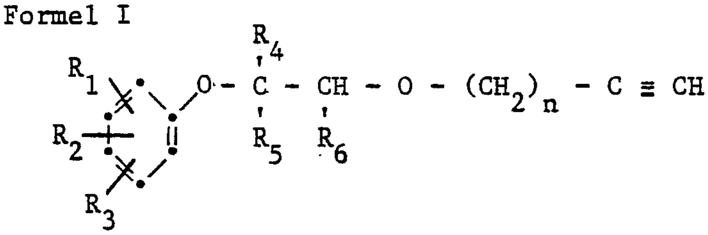

(I),

worin

$R_1$ Wasserstoff, $C_1$-$C_4$-Alkyl, Halogen, $C_1$-$C_4$-Alkoxy, $C_3$-$C_4$-Alkenyloxy, $C_3$-$C_5$-Alkinyloxy, Trifluormethyl, Nitro oder Cyano;

$R_2$ Wasserstoff, $C_1$-$C_4$-Alkyl, Halogen, $C_1$-$C_4$-Alkoxy, Trifluormethyl, Nitro oder zusammen mit $R_1$ Methylendioxy;

$R_3$ Wasserstoff, $C_1$-$C_4$-Alkyl oder Halogen;

$R_4$ Wasserstoff oder $C_1$-$C_4$-Alkyl;

$R_5$ Wasserstoff oder $C_1$-$C_4$-Alkyl;

$R_6$ Wasserstoff, $C_1$-$C_4$-Alkyl oder zusammen mit $R_5$ die $-(CH_2)_3-$ oder $-(CH_2)_4-$ Gruppe und

n eine der Zahlen 1, 2 oder 3 bedeuten.

Wegen ihrer Wirkung als Schädlingsbekämpfungsmittel bevorzugt sind erfindungsgemäss Verbindungen der Formel I, die dadurch gekennzeichnet sind, dass

$R_1$ Wasserstoff, $C_1$-$C_4$-Alkyl, Halogen, Methoxy, Aethoxy, Allyloxy, $C_3$-$C_4$-Alkinyloxy, Trifluormethyl oder Nitro;
$R_2$ Wasserstoff, Methyl, Halogen, Methoxy oder zusammen mit $R_1$ 3,4-Methylendioxy;
$R_3$ Wasserstoff, Methyl, Chlor oder Fluor;
$R_4$ Wasserstoff oder $C_1$-$C_4$-Alkyl;
$R_5$ Wasserstoff oder Methyl;
$R_6$ Wasserstoff, Methyl, Aethyl oder zusammen mit $R_5$ die $-(CH_2)_4$ Gruppe und
n eine der Zahlen 1, 2 oder 3 bedeuten.

Hervorzuheben sind auch diejenigen Verbindungen der Formel I, worin

$R_1$ Wasserstoff, $C_1$-$C_4$-Alkyl, Chlor, Brom, Methoxy, Propargyloxy, Trifluormethyl oder Nitro;
$R_2$ und $R_3$ Wasserstoff, Methyl oder Chlor;
$R_4$ Wasserstoff oder Methyl;
$R_5$ Wasserstoff;
$R_6$ Wasserstoff, Methyl, Aethyl oder zusammen mit $R_5$ die $-(CH_2)_4$ Gruppe und
n eine der Zahlen 1 oder 3 bedeuten.

Eine bevorzugte Gruppe von Verbindungen der Formel I ist dadurch gekennzeichnet, dass n die Zahl 1 bedeutet.

Besonders wirksam sind erfindungsgemässe Verbindungen der Formel I, worin

$R_1$ Wasserstoff oder in der 2- oder 4-Stellung befindliches Chlor, Brom oder Propargyloxy;
$R_2$ und $R_3$ Wasserstoff oder Chlor;

$R_4$ und $R_5$ Wasserstoff;
$R_6$ Wasserstoff, Methyl oder Aethyl und
n die Zahl 1 bedeuten.

Die Verbindungen der Formel I können nach an sich bekannten Verfahren hergestellt werden (vgl. u.a. "Methoden d. organischen Chemie", Houben-Weyl, Bd. VI/3, S. 24 ff, 1965; C.A.Buchler, D.E. Pearson," Survey of Organic Syntheses", Ch. 6, p. 285 ff, 1970; W.P.Weber, G.W. Gokel, "Phase Transfer Catalysis in Organic Syntheses", Ch. 5, p. 77, 1977).

So kann man z.B. die Verbindungen der Formel I mit Hilfe der bekannten Aether-Synthese nach Williamson herstellen:

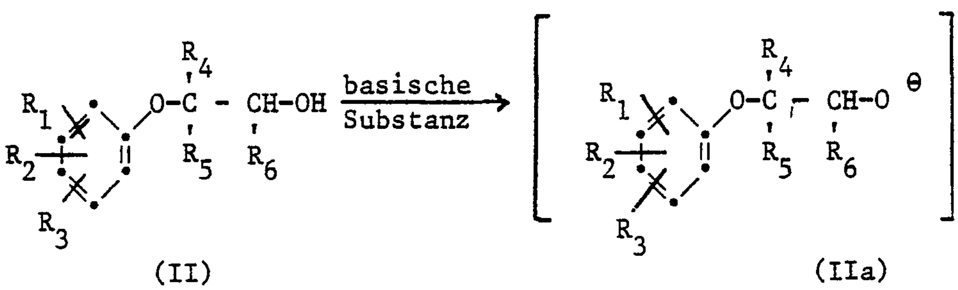


(II) (IIa)

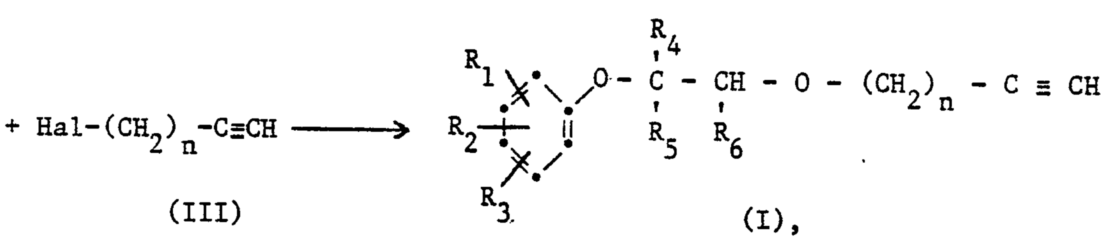


(III) (I),

Bei dieser Synthese werden zur Bildung des Alkoholats der Formel IIa basische Substanzen, wie Alkalimetallalkoholate niederer Alkohole, Natriumhydrid, Natriumamid, Alkalimetalle oder Alkalihydroxyde, z.B. NaOH, KOH, verwendet. Gegebenenfalls kann als Katalysator ein Alkalimetalljodid, z.B. Kaliumjodid, eingesetzt werden. Die Umsetzung wird im allgemeinen unter Normaldruck und bei einer Temperatur von 20-150°C, vorzugsweise 40-120°C, in Gegenwart von aprotischen

BAD ORIGINAL

Lösungsmitteln, z.B. Kohlenwasserstoffen, wie Benzol oder Toluol, höhersiedenden Aethern, wie Dioxan oder Tetrahydrofuran, oder reaktionsfördernden Lösungsmittel, wie Acetonitril, Dimethylsulfoxid, Hexamethylphosphorsäuretriamid usw. durchgeführt.

Die Verbindungen der Formel I lassen sich auch mittels der sogenannten "Phasentransfer-Katalyse" herstellen, und zwar durch Umsetzung einer Verbindung der Formel II mit aktivierten Alkinylhalogeniden der Formel III, z.B. Propargylbromid:

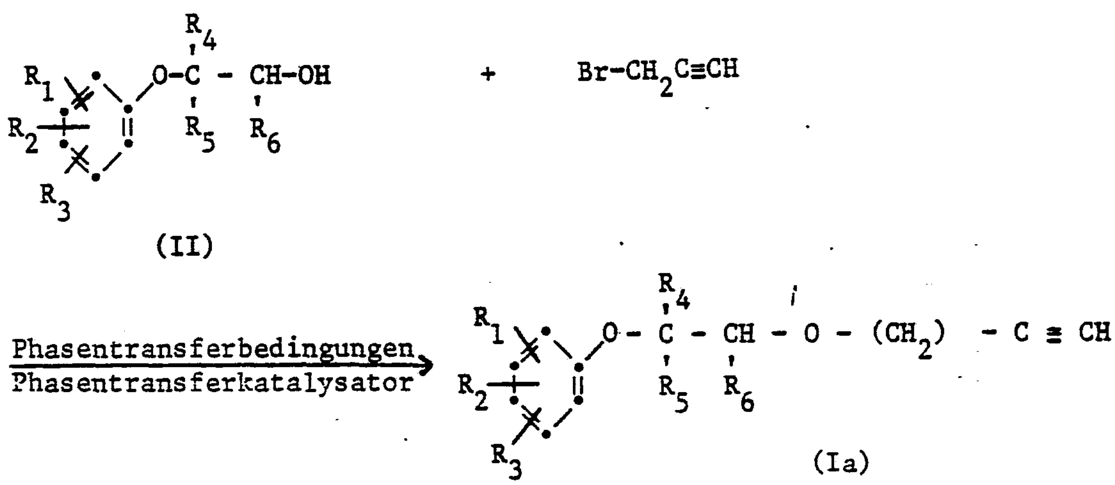


(II) (Ia)

Diese Umsetzung wird im allgemeinen unter Normaldruck und bei einer Reaktionstemperatur, die zwischen Raumtemperatur und 80°C, vorzugsweise zwischen 20 und 60°C, liegt, durchgeführt, wobei ein Alkalimetallhydroxyd, z.B. Natriumhydroxyd, als Kondensationsmittel dient. Als Katalysatoren kommen die bekannten Phasentransfer-Katalysatoren, vorzugsweise Tetrabutylammonium-bisulfat, in Betracht. Die Lösungsmittel-Kombinationen für die Phasentransfer-Reaktion bestehen aus einer wässrigen und einer mit Wasser nicht mischbaren Phase, z.B. 50%-iger wässriger Natriumhydroxid-Lösung und Methylenchlorid.

In den vorstehenden Formeln Ia, II, IIa und III haben die Reste $R_1$, $R_2$, $R_3$, $R_4$, $R_5$ und $R_6$ sowie n die für Formel I angegebenen Bedeutungen, und Hal bedeutet Chlor oder Brom.

Die als Ausgangsstoffe verwendeten Verbindungen der Formeln II und III sind bekannt oder können analog bekannten Verfahren erhalten werden. Beispielsweise können die gegebenenfalls substituierten Phenoxyäthanol-Derivate der Formel II entsprechend den in folgenden Schrifttumstellen aufgeführten Arbeitsweisen hergestellt werden: "Methoden der org. Chemie" (Houben-Weyl) Bd. VI/3, (1965); W.W.Carlson et al. J. Amer. Chem. Soc. 69, p.1952 (1947); Deutsche Offenlegungsschrift Nr. 2,654,646; US-Patent Nr. 3,452,081.

Aus der US-Patentschrift Nr. 4.017.549 sind 1-(4-Benzyl)-phenoxy-3-alkinyloxy-propane und 1-(4-Phenylthio)-phenoxy-3-alkinyloxy-propane bekannt, die als Insekten-Juvenilhormone wirksam sind. Ferner werden in der deutschen Offenlegungsschrift Nr. 28 03 806 α-Phenoxy-ω-halogenalkinyloxy-alkane und in der DDR-Patentschrift Nr. 103 543 substituierte 1-Phenoxy-2-alkoxy-äthane mit Juvenilhormon-Wirksamkeit beschrieben.

Ueberraschenderweise wurde nun gefunden, dass die erfindungsgemässen 1-Phenoxy-2-alkinyloxy-äthane der Formel I bei guter Pflanzenverträglichkeit und geringer Warmblütertoxizität ausgezeichnete Wirksamkeit als Schädlingsbekämpfungsmittel, vor allem als Kontaktinsektizide und Frassgifte zur Bekämpfung von Pflanzen und Tiere befallenden Insekten sowie ihrer larvalen Stadien und Eier, aufweisen.

Insbesondere eignen sich die Verbindungen der Formel I zur Bekämpfung von Insekten der Familien: Acrididae, Blattidae, Gryllidae, Gryllotalpidae, Tettigoniidae, Cimicidae, Phyrrhocoridae, Reduviidae, Aphididae, Delphacidae, Diaspididae, Pseudococcidae, Chrysomelidae, Coccinellidae, Bruchidae, Scarabaeidae, Dermestidae, Tenebrionidae, Curculionidae, Tineidae, Noctuidae, Lymantriidae, Pyralidae, Galleridae, Culicidae, Tipulidae, Stomoxydae, Muscidae, Calliphoridae, Trypetidae und Pulicidae.

Neben ihrer sehr günstigen Wirkung gegenüber Fliegen, wie z.B. Musca domestica, und Mückenlarven eignen sich Verbindungen der Formel I auch zur Bekämpfung von pflanzenschädigenden Frassinsekten in Zier- und Nutzpflanzungen, insbesondere in Baumwollkulturen (z.B. Spodoptera littoralis und Heliothis virescens) sowie in Gemüsekulturen (z.B. Leptinotarsa decemlineata und Myzus persicae).

Die Verbindungen der Formel I sind weiterhin zur Bekämpfung von Ektoparasiten an Haus- und Nutztieren, z.B., durch Tier-, Stall- und Weidebehandlung geeignet.

Hervorzuheben ist auch die ausgeprägte ovizide bzw. ovolarvizide Wirksamkeit der erfindungsgemässen Verbindungen der Formel I, die sich auf Eier und Eigelege von verschiedenartigen, insbesondere Pflanzen und Tiere befallenden Schadinsekten erstreckt.

Die erfindungsgemässen Verbindungen der Formel I sind ferner als Synergisten in Kombination mit anderen bekannten Insektiziden vom Phosphorsäureester-, Chlorkohlenwasserstoff-, Carbamat- und Pyrethroid-Typ, insbesondere gegenüber resistenten Insektenstämmen, wirksam. Ueberraschenderweise ist die insektizide Wirksamkeit der genannten Wirkstoffkombinationen wesentlich höher als die Summe der Wirkungen der einzelnen Wirkstoffe. Es liegt demnach ein echter synergistischer Effekt vor und nicht nur eine Wirkungsergänzung oder -Summierung. Dieser synergistische Effekt tritt besonders stark bei bestimmten Konzentrationsverhältnissen hervor. Im Rahmen der vorliegenden Erfindung hat sich ein Gew.-Verhältnis von bekanntem Wirkstoff zu einer synergistisch wirksamen Verbindung der Formel I von 9:1 bis 1:9, vorzugsweise 4:1 bis 1:4, als günstig erwiesen. Die erfindungsgemässen synergistischen Wirkstoffkombinationen zeichnen sich dadurch aus, dass schon bei geringer Dosierung der angestrebte Effekt eintritt, was vom Standpunkt der Toxikologie und Oekologie einen erheblichen Vorteil darstellt. Von besonderem Interesse erweist sich die Anwendung der erfindungsgemässen synergistischen

Wirkstoffkombinationen bei der Bekämpfung resistenter und auch multiresistenter Insektenstämme. Es handelt sich hierbei in der Hauptsache um Resistenz gegenüber Phosphorsäureestern, Carbamaten und pyrethroiden Verbindungen. Derartige resistente Stämme konnten mit den bisher üblichen Insektiziden nur schwierig, bzw. nur unter Einsatz bedenklich hoher Wirkstoffdosierungen bekämpft werden.

Unter den Verbindungen der Formel I hat sich das 1-(4'-Propargyloxy)-phenoxy-2-(propargyloxy)-äthan als besonders wirksamer Synergist erwiesen. Gute synergistische Wirkungen zeigen auch 1-(2',4'-Dichlor)-phenoxy-2-(propargyloxy)-butan, 1-(4'-Propargyloxy)-phenoxy-2-(propargyloxy)-butan und 1-(2',6'-Dichlor)-phenoxy-2-(propargyloxy)-butan.

Abgesehen von ihrem synergistischen Effekt lässt sich die Wirkung der erfindungsgemässen Verbindungen bzw. der die enthaltenden Mittel durch Zusatz von anderen Insektiziden und/oder Akariziden wesentlich verbreitern und an gegebene Umstände anpassen.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden.

Die Herstellung erfindungsgemässer Mittel erfolgt in an sich bekannter Weise durch inniges Vermischen und/oder Vermahlen von Wirkstoffen der Formel I mit den geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate: Spritzpulver (wettable powders), Pasten, Emulsionen;

b) Lösungen.

Der Gehalt an Wirkstoff in den oben beschriebenen Mitteln liegt zwischen 0,1 bis 95%.

Die Wirkstoffe der Formel I können beispielsweise wie folgt formuliert werden:

Stäubemittel: Zur Herstellung eines a) 5%igen und b) 2%igen Stäubemittels werden die folgenden Stoffe verwendet:

a) 5 Teile Wirkstoff,
95 Teile Talkum;

b) 2 Teile Wirkstoff,
1 Teil hochdisperse Kieselsäure,
97 Teile Talkum.

Die Wirkstoffe werden mit den Trägerstoffen vermischt und vermahlen.

Granulat: Zur Herstellung eines 5%igen Granulates werden die folgenden Stoffe verwendet:

5,00 Teile Wirkstoff,
0,25 Teile Epichlorhydrin,
0,25 Teile Cetylpolyglykoläther,
3,50 Teile Polyäthylenglykol,
91,00 Teile Kaolin (Korngrösse 0,3 - 0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40%igen, b) und c) 25%igen d) 10%igen Spritzpulvers werden folgende Bestandteile verwendet:

| | | |
|---|---|---|
| a) | 40 | Teile Wirkstoff, |
| | 5 | Teile Ligninsulfonsäure-Natriumsalz, |
| | 1 | Teil Dibutylnaphthalinsulfonsäure-Natriumsalz, |
| | 54 | Teile Kieselsäure; |
| b) | 25,0 | Teile Wirkstoff, |
| | 4,5 | Teile Calcium-Ligninsulfonat, |
| | 1,9 | Teile Champagne-Kreide/Hydroxyäthylcellulose-Gemisch (1:1), |
| | 1,5 | Teile Natrium-dibutyl-naphthalinsulfonat, |
| | 19,5 | Teile Kieselsäure, |
| | 19,5 | Teile Champagne-Kreide, |
| | 28,1 | Teile Kaolin; |
| c) | 25,0 | Teile Wirkstoff, |
| | 2,5 | Teile Isooctylphenoxy-polyoxyäthylen-äthanol, |
| | 1,7 | Teile Champagne-Kreide/Hydroxyäthylcellulose-Gemisch (1:1), |
| | 8,3 | Teile Natriumaluminiumsilikat, |
| | 16,5 | Teile Kieselgur, |
| | 46,0 | Teile Kaolin; |

d) 10 Teile Wirkstoff,
3 Teile Gemisch der Natriumsalz von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentration verdünnen lassen.

<u>Emulgierbare Konzentrate:</u> Zur Herstellung eines a) 10%igen b) 25%igen und c) 50%igen emulgierbaren Konzentrates werden folgende Stoffe verwendet:

a) 10,0 Teile Wirkstoff,
3,4 Teile epoxydiertes Pflanzenöl,
3,4 Teile eines Kombinationsemulgators, bestehend aus Fettalkoholpolyglykoläther und Alkylaralkylsulfonat-Calcium-Salz,
40,0 Teile Dimethylformamid,
43,2 Teile Xylol;

b) 25,0 Teile Wirkstoff,
2,5 Teile epoxydiertes Pflanzenöl,
10,0 Teile eines Alkylarylsulfonat/Fettalkoholpolyglykoläther-Gemisches,
5,0 Teile Dimethylformamid,
57,5 Teile Xylol,

| | | |
|---|---|---|
| c) | 50,0 | Teile Wirkstoff, |
| | 4,2 | Teile Tributylphenol-Polyglykoläther, |
| | 5,8 | Teil Calcium-Dodecylbenzolsulfonat, |
| | 20,0 | Teile Cyclohexanon, |
| | 20,0 | Teile Xylol. |

Aus solchen Konzentrationen können durch Verdünnen mit Wasser Emulsionen jeder gewünschten Konzentration hergestellt werden.

Sprühmittel: Zur Herstellung eines a) 5%igen und b) 95%igen Sprühmittels werden die folgenden Bestandteile verwendet:

| | | |
|---|---|---|
| a) | 5 | Teile Wirkstoff, |
| | 1 | Teil Epichlorhydrin, |
| | 94 | Teile Benzin (Siedegrenzen 160-190°C); |

| | | |
|---|---|---|
| b) | 95 | Teile Wirkstoff, |
| | 5 | Teile Epichlorhydrin. |

Beispiel 1

1-(3',4'-Dimethyl)-phenoxy-2-(1"-pent-4"-inyloxy)-propan

5,22 g einer Suspension (50%) von Natriumhydrid in Paraffinöl werden zu einem Gemisch aus 180 ml abs. Tetrahydrofuran und 30 ml Hexamethylphosphorsäuretriamid (HMTP) gegeben. Unter Stickstoff werden 18 g 1-(3',4'-Dimethyl)-phenoxy-propan-2-ol, gelöst in 90 ml Tetrahydrofuran, unter starkem Rühren zugetropft. Das Reaktionsgemisch wird anschliessend während 2 Std. auf 50°C gehalten. Nun werden 10,7 g 5-Chlor-1-pentin, gelöst in 10 ml Tetrahydrofuran während 20 Min. zugetropft. Man gibt weitere 30 ml HMTP zu und hält das Gemisch unter starkem Rühren für 16 Std. bei 50-60°C. Nach dem Abkühlen wird das Reaktionsgemisch auf eine Mischung aus 500 ml gesättigter Kochsalzlösung und 500 g Eis gegossen und mit 1000 ml Aether extrahiert. Die ätherische Lösung wird mit 2-N Natriumhydroxid gewaschen und dann mit gesättigter Kochsalzlösung neutral gewaschen. Die ätherische Lösung wird mit Natriumsulfat getrocknet und der Aether am Vakuum abgesaugt. Fraktionierte Destillation am Hochvakuum ergibt reines 1-(3',4'-Dimethyl)-phenoxy-2-(1"-pent-4"-inyloxy)-propan, Sdp. 101-103°/0,09 Torr.

Das Ausgangsprodukt kann wie folgt erhalten werden:

3-(3',4'-Dimethyl)-phenoxy-propan-2-ol

Ein Gemisch aus 12,2 g 3,4-Dimethylphenol, 11,6 g Propylenoxid und 0,4 g Triäthylamin werden während 16 Std. auf 110°C erwärmt. Nach dem Abkühlen wird in 1000 ml Chloroform aufgenommen, mit gesättigter Kochsalzlösung und dann mit Natriumbicarbonat-Lösung gewaschen. Nach dem Trocknen der Lösung mit Natriumsulfat wird das Chloroform am Vakuum abgesaugt. Das rohe Produkt wird durch fraktionierte Destillation gereinigt, Sdp. 159°C/13 Torr.

Beispiel 2

1-(4'-Chlor)-phenoxy-2-propargyloxy)-äthan

Zu einem Gemisch aus 40 ml einer 50%igen wässrigen Natriumhydroxid-Lösung und 100 ml Methylenchlorid werden 17,2 g 2-(4'-Chlor)-phenoxy-äthanol und 1,7 g Tetrabutylammoniumbisulfat gegeben. Unter sehr starkem Rühren werden bei Raumtemperatur 18 g Propargylbromid zugetropft. Man erwärmt das Gemisch auf Rückflusstemperatur und lässt es 16 Std. unter sehr starkem Rühren reagieren. Das abgekühlte Reaktionsgemisch wird in 1000 ml Aether aufgenommen und mit gesättigter Kochsalzlösung neutral gewaschen. Die ätherische Lösung wird mit Natriumsulfat getrocknet und der Aether abgesaugt. Das rohe Produkt wird durch fraktionierte Destillation am Hochvakuum gereinigt; Sdp. 90-93°C/0,1 Torr.

Das Ausgangsprodukt kann wie folgt hergestellt werden:

2-(4'-Chlor)-phenoxy-äthanol

25,6 g 4-Chlorphenol, 17,6 g Aethylencarbonat und 7,6 g Tetrabutylammoniumbromid werden vermischt und während 3 Std. auf 150°C erhitzt. Das Reaktionsgemisch wird anschliessend weitere 16 Std. bei 150°C gehalten. Nach dem Abkühlen wird in 1000 ml Chloroform aufgenommen, unter Eiskühlung mit 2-N Natriumhydroxid und anschliessend mit gesättigter Kochsalzlösung gewaschen. Man trocknet die Chloroformlösung mit Natriumsulfat und saugt das Chloroform am Vakuum ab. Durch fraktionierte Destillation erhält man 2'-(4'-Chlor)-phenoxy-äthanol; Sdp. 154-155°C/14 Torr.

Analog den in den vorstehenden Beispielen beschriebenen Arbeitsweise werden die folgenden Verbindungen der Formel I hergestellt:

| $R_1$ | $R_2$ | $R_3$ | $R_4$ | $R_5$ | $R_6$ | n | Physikalische Daten |
|---|---|---|---|---|---|---|---|
| $3-C_2H_5$ | H | H | H | H | H | 1 | $n_D^{21}$ : 1.5142 |
| $2-C_2H_5$ | H | H | H | H | H | 1 | $n_D^{19}$ : 1.5142 |
| $4-CH_3$ | H | H | H | H | H | 1 | $n_D^{19}$ : 1.5194 |
| $2-CH_3$ | H | H | H | H | H | 1 | Sdp. 94-95°C/0,5 Torr |
| 4-Cl | H | H | H | H | H | 1 | Sdp. 90-93°C/0,1 Torr |
| 4-Br | H | H | H | H | H | 1 | Sdp. 110-111°C/0,2 Torr |
| 4-F | H | H | H | H | H | 1 | Sdp. 73-76°C/0,07 Torr |
| $4-i-C_3H_7$ | H | H | H | H | H | 1 | Sdp. 87-90°C/0,07 Torr |
| $2-i-C_3H_7$ | H | H | H | H | H | 1 | Sdp. 82-85°C/0,06 Torr |
| $4-s-C_4H_9$ | H | H | H | H | H | 1 | Sdp. 94-96°C/0,05 Torr |
| $3-CH_3$ | H | H | H | H | H | 1 | Sdp. 76-79°C/0,04 Torr |
| $2-s-C_4H_9$ | H | H | H | H | H | 1 | Sdp. 82-84°C/0,04 Torr |

| $R_1$ | $R_2$ | $R_3$ | $R_4$ | $R_5$ | $R_6$ | n | Physikalische Daten |
|---|---|---|---|---|---|---|---|
| 2-Cl | 4-Cl | H | H | H | H | 1 | Sdp.102-104°C/0,04 Torr |
| 2-Cl | 6-Cl | H | H | H | H | 1 | Sdp. 90°C/0,04 Torr |
| 3-Cl | 4-Cl | 5-Cl | H | H | H | 1 | Smp. 52-53°C |
| 2-Cl | 4-Cl | 5-Cl | H | H | H | 1 | Sdp.120-123°C/0,04 Torr |
| H | H | H | H | H | H | 1 | Sdp. 66-67°C/0,04 Torr |
| 3-$OCH_3$ | 4-$OCH_3$ | H | H | H | H | 1 | Smp. 65-67°C |
| 4-$NO_2$ | H | H | H | H | H | 1 | Smp. 92-94°C |
| 3,4 - $OCH_2O$ - | | H | H | H | H | 1 | Sdp. 113-116°C/0,01 Torr |
| 4-$HC{\equiv}CCH_2O$ | H | H | H | H | H | 1 | Smp. 48-50°C |
| 2-Cl | 4-Cl | 6-Cl | H | H | H | 1 | Sdp. 103-106°C/0,12 Torr |
| 3-$CF_3$ | H | H | H | H | H | 1 | Sdp. 80°C/0,4 Torr |
| 2-$CH_3$ | 4-$CH_3$ | H | H | H | H | 1 | Sdp. 85-87°C/0,06 Torr |

| $R_1$ | $R_2$ | $R_3$ | $R_4$ | $R_5$ | $R_6$ | n | Physikalische Daten |
|---|---|---|---|---|---|---|---|
| 3-$CH_3$ | 4-$CH_3$ | H | H | H | H | 1 | Sdp. 105°C/0,4 Torr |
| 2-$CH_3$ | 3-$CH_3$ | H | H | H | H | 1 | Sdp. 86-87°C/0,2 Torr |
| 2-$CH_3$ | 5-$CH_3$ | H | H | H | H | 1 | Sdp. 79-80°C/0,15 Torr |
| 2-$CH_3$ | 6-$CH_3$ | H | H | H | H | 1 | Sdp. 79-80°C/0,2 Torr |
| 3-$CH_3$ | 5-$CH_3$ | H | H | H | H | 1 | Sdp. 88°C/0,15 Torr |
| 2-$CH_3$ | 4-$CH_3$ | H | H | H | H | 1 | Sdp. 96-97°C/0,08 Torr |
| 2-$CH_3$ | 3-$CH_3$ | 6-$CH_3$ | H | H | H | 1 | Sdp. 93-94°C/0,13 Torr |
| 4-t-$C_4H_9$ | H | H | H | H | H | 1 | Sdp. 93-95°C/0,04 Torr |
| 4-$CH_3$ | H | H | H | H | $CH_3$ | 1 | Sdp. 68-75°C/0,06 Torr |
| 2-$CH_3$ | H | H | H | H | $CH_3$ | 1 | Sdp. 70-71°C/0,05 Torr |
| 3-$CH_3$ | H | H | H | H | $CH_3$ | 1 | Sdp. 66-69°C/0,04 Torr |
| 2-$C_2H_5$ | H | H | H | H | $CH_3$ | 1 | Sdp. 69-72°C/0,04 Torr |

| $R_1$ | $R_2$ | $R_3$ | $R_4$ | $R_5$ | $R_6$ | n | Physikalische Daten |
|---|---|---|---|---|---|---|---|
| 3-$C_2H_5$ | H | H | H | H | $CH_3$ | 1 | Sdp. 78-79°C/0,06 Torr |
| 4-Cl | H | H | H | H | $CH_3$ | 1 | Sdp. 78-80°C/0,05 Torr |
| 4-$C_2H_5$ | H | H | H | H | $CH_3$ | 1 | Sdp. 79-81°C/0,06 Torr |
| 4-Br | H | H | H | H | $CH_3$ | 1 | Sdp. 92-94°C/0,08 Torr |
| 2-i-$C_3H_7$ | H | H | H | H | $CH_3$ | 1 | Sdp. 76°C/0,06 Torr |
| 2-Cl | 4-Cl | H | H | H | $CH_3$ | 1 | Sdp. 102-104°C/0,07 Torr |
| 3-Cl | 4-Cl | 5-Cl | H | H | $CH_3$ | 1 | Sdp. 125-129°C/0,25 Torr |
| 4-F | H | H | H | H | $CH_3$ | 1 | Sdp. 71-75°C/0,25 Torr |
| 4-i-$C_3H_7$ | H | H | H | H | $CH_3$ | 1 | Sdp. 151-153°C/12 Torr |
| 4-s-$C_4H_9$ | H | H | H | H | $CH_3$ | 1 | Sdp. 89-91°C/0,8 Torr |
| 4-t-$C_4H_9$ | H | H | H | H | $CH_3$ | 1 | Sdp. 114-117°C/0,2 Torr |
| 2-s-$C_4H_9$ | H | H | H | H | $CH_3$ | 1 | Sdp. 100°C/0,2 Torr |

| $R_1$ | $R_2$ | $R_3$ | $R_4$ | $R_5$ | $R_6$ | n | Physikalische Daten |
|---|---|---|---|---|---|---|---|
| 2-Cl | 6-Cl | H | H | H | $CH_3$ | 1 | Sdp. 107°C/0,2 Torr |
| 2-Cl | 4-Cl | 5-Cl | H | H | $CH_3$ | 1 | Sdp. 118-119°C/0,1 Torr |
| H | H | H | H | H | $CH_3$ | 1 | Sdp. 68-69°C/0,18 Torr |
| 3-$CF_3$ | H | H | H | H | $CH_3$ | 1 | Sdp. 71°C/0,15 Torr |
| 2-Cl | 4-Cl | 6-Cl | H | H | $CH_3$ | 1 | Sdp. 118-119°C/0,2 Torr |
| 2-$CH_3$ | 4-$CH_3$ | H | H | H | $CH_3$ | 1 | Sdp. 96-97°C/0,5 Torr |
| 4-HC≡$CCH_2O$ | H | H | H | H | $CH_3$ | 1 | Sdp. 125-127°C/0,25 Torr |
| 2-$CH_3$ | 5-$CH_3$ | H | H | H | $CH_3$ | 1 | Sdp. 87-88°C/0,28 Torr |
| 2-$CH_3$ | 3-$CH_3$ | H | H | H | $CH_3$ | 1 | Sdp. 94-97°C/0,2 Torr |
| 3-$CH_3$ | 4-$CH_3$ | H | H | H | $CH_3$ | 1 | Sdp. 86-88°C/0,15 Torr |
| 3-$CH_3$ | 5-$CH_3$ | H | H | H | $CH_3$ | 1 | Sdp. 89°C/0,2 Torr |
| 2-$CH_3$ | 3-$CH_3$ | 5-$CH_3$ | H | H | $CH_3$ | 1 | Sdp. 88-90°C/0,12 Torr |

| $R_1$ | $R_2$ | $R_3$ | $R_4$ | $R_5$ | $R_6$ | n | Physikalische Daten |
|---|---|---|---|---|---|---|---|
| 2-$CH_3$ | 3-$CH_3$ | 6-$CH_3$ | H | H | $CH_3$ | 1 | Sdp. 95-97°C/0,18 Torr |
| 4-$CH_3$ | H | H | H | H | $C_2H_5$ | 1 | Sdp. 77-79°C/0,09 Torr |
| 4-$C_2H_5$ | H | H | H | H | $C_2H_5$ | 1 | Sdp. 87-88°C/0,09 Torr |
| 4-HC≡$CCH_2O$ | H | H | H | H | $C_2H_5$ | 1 | Sdp. 134-137°C/0,18 Torr |
| 4-t-$C_4H_9$ | H | H | H | H | $C_2H_5$ | 1 | Sdp. 97-98°C/0,05 Torr |
| 2-Cl | 4-Cl | H | H | H | $C_2H_5$ | 1 | Sdp. 103°C/0,06 Torr |
| 2-Cl | 6-Cl | H | H | H | $C_2H_5$ | 1 | Sdp. 89-90°C/0,05 Torr |
| 2-Cl | 4-Cl | 5-Cl | H | H | $C_2H_5$ | 1 | Sdp. 124-125°C/0,09 Torr |
| 2-Cl | 4-Cl | 6-Cl | H | H | $C_2H_5$ | 1 | Sdp. 106°C/0,04 Torr |
| 4-HC≡$CCH_2O$ | H | H | H | $R^5+R^6$ =$-(CH_2)_4-$ | | 1 | Sdp. 145-146°C/0,35 Torr $n_D^{19}$: 1.5351 |
| 4-Cl | H | H | H | $R^5+R^6$ =$-(CH_2)_4-$ | | 1 | $n_D^{19}$ : 1.5368 |

| $R_1$ | $R_2$ | $R_3$ | $R_4$ | $R_5$ | $R_6$ | n | Physikalische Daten |
|---|---|---|---|---|---|---|---|
| 2-Cl | 6-Cl | H | H | $R^5+R^6 = -(CH_2)_4-$ | | 1 | $n_D^{19}$ : 1.5445 |
| $3-C_2H_5$ | H | H | H | H | H | 3 | Sdp. 172-175°C/0,05 Torr |
| H | H | H | H | H | H | 3 | Sdp. 83°C/0,09 Torr |
| $2-CH_3$ | $6-CH_3$ | H | H | H | H | 3 | Sdp. 87-88°C/0,08 Torr |
| $2-CH_3$ | $3-CH_3$ | H | H | H | H | 3 | Sdp. 106-108°C/0,2 Torr |
| $2-CH_3$ | $4-CH_3$ | H | H | H | H | 3 | Sdp. 96-97°C/0,08 Torr |
| $2-CH_3$ | $5-CH_3$ | H | H | H | H | 3 | Sdp. 102-103°C/0,2 Torr |
| $3-CH_3$ | $4-CH_3$ | H | H | H | H | 3 | Sdp. 100-102°C/0,1 Torr |
| $2-CH_3$ | $4-CH_3$ | H | H | H | $CH_3$ | 3 | Sdp. 93-95°C/0,13 Torr |
| $2-CH_3$ | $3-CH_3$ | H | H | H | $CH_3$ | 3 | Sdp. 102-104°C/0,2 Torr |
| $3-CH_3$ | $4-CH_3$ | H | H | H | $CH_3$ | 3 | Sdp. 101-103°C/0,09 Torr |
| $2-CH_3$ | $5-CH_3$ | H | H | H | $CH_3$ | 3 | $n_D^{20}$ : 1.4967 |

| $R_1$ | $R_2$ | $R_3$ | $R_4$ | $R_5$ | $R_6$ | n | Physikalische Daten |
|---|---|---|---|---|---|---|---|
| 2-Cl | 4-Cl | H | H | H | $C_2H_5$ | 3 | Smp. 78-79°C |
| 3-Cl | 4-Cl | 5-Cl | H | H | $C_2H_5$ | 3 | $n_D^{19}$ : 1.5340 |
| 2-Cl | 4-Cl | H | $CH_3$ | $CH_3$ | H | 1 | $n_D^{20}$ : 1.5280 |
| 3,4 - $OCH_2O$- | | H | $CH_3$ | $CH_3$ | H | 1 | $n_D^{20}$ : 1.5240 |
| 4-Cl | H | H | $CH_3$ | $CH_3$ | H | 1 | $n_D^{20}$ : 1.5177 |
| 4-Br | H | H | $CH_3$ | $CH_3$ | H | 1 | $n_D^{20}$ : 1.5333 |
| H | H | H | $CH_3$ | $CH_3$ | H | 1 | $n_D^{20}$ : 1.5055 |
| 4-$C_2H_5$ | H | H | $CH_3$ | H | H | 1 | Sdp. 111-115°C/0,5 Torr |
| 2-Cl | 4-Cl | H | $CH_3$ | H | H | 1 | $n_D^{20}$ : 1.5328 |
| 4-Cl | H | H | $CH_3$ | H | H | 1 | $n_D^{20}$ : 1.5245 |
| H | H | H | $CH_3$ | H | H | 1 | $n_D^{20}$ : 1.5122 |

| $R_1$ | $R_2$ | $R_3$ | $R_4$ | $R_5$ | $R_6$ | n | Physikalische Daten |
|---|---|---|---|---|---|---|---|
| $4-H_2C=CHCH_2O$ | H | H | H | H | $CH_3$ | 1 | |
| $4-C_2H_5$ | H | H | H | H | H | 1 | |
| $4-H_3CC≡CCH_2O$ | H | H | $CH_3$ | $CH_3$ | H | 1 | |
| $4-HC≡CCH_2CH_2O$ | H | H | H | H | H | 1 | |
| 4-Cl | H | H | H | H | H | 2 | |
| $4-HC≡CCH_2O$ | H | H | H | H | H | 2 | |
| $4-HC≡C-CH_2O$ | H | H | $CH_3$ | H | H | 1 | Smp. 60,5 - 62,5°C |
| 4-Cl | H | H | $CH_3$ | H | $CH_3$ | 1 | $n_D^{20}$ : 1.5181 |
| $2-CH_3$ | $3-CH_3$ | H | $CH_3$ | H | $CH_3$ | 1 | $n_D^{20}$ : 1.5093 |
| $4-HC≡C-CH_2O$ | H | H | $CH_3$ | H | $CH_3$ | 1 | $n_D^{20}$ : 1.5220 |
| H | H | H | $CH_3$ | H | $CH_3$ | 1 | $n_D^{20}$ : 1.5089 |
| 2-Cl | 4-Cl | H | $CH_3$ | H | $CH_3$ | 1 | $n_D^{20}$ : 1.5912 |

| $R_1$ | $R_2$ | $R_3$ | $R_4$ | $R_5$ | $R_6$ | n | Physikalische Daten |
|---|---|---|---|---|---|---|---|
| 3-$CH_3$ | 4-Cl | H | $CH_3$ | H | $CH_3$ | 1 | $n_D^{20}$ : 1.5198 |
| 4-Cl | H | H | $C_2H_5$ | H | H | 1 | $n_D^{20}$ : 1.5190 |
| 4-HC≡C-$CH_2$O | H | H | $C_2H_5$ | H | H | 1 | $n_D^{20}$ : 1.5230 |

Beispiel 3

Kontaktwirkung auf Musca domestica:

In jeweils eine Petrischale (9 cm Durchmesser) mit einer geätzten Innenfläche wurde 1 ml einer acetonischen Lösung enthaltend 1 bzw. 5 mg des zu prüfenden Wirkstoffes pipettiert. Die Wirkstofflösung wurde gleichmässig auf dem Schalenboden verteilt, wobei das Aceton verdampfte. Eine Stunde nach Verdampfen des Acetons wurden pro behandelte Schale 10 drei Tage alte, in Kältestarre befindliche, normalsensible adulte Fliegen (Musca domestica)angesetzt. Die Ansätze wurden bei Raumtemperatur gehalten.

Die Auswertung erfolgte aufgrund des Prozentsatzes der durch den Kontakt mit der behandelten Unterlage abgetöteten Tiere (% Rückenlage).

Verbindungen gemäss den vorstehenden Beispielen 1 und 2 zeigten gute Wirkung in obigem Test.

Beispiel 4

Kontaktwirkung auf Aedes aegypti (Larven):

Mittels einer Pipette wurde 1 ml einer acetonischen Lösung der zu prüfenden Wirksubstanz auf die Oberfläche von 100 ml in einem Plastikbecher befindlichem Wasser gegeben. Die acetonische Lösung enthielt die Wirksubstanz in einer solchen Menge, dass sich Wirkstoffkonzentrationen von 10 bzw. 1 ppm in dem im jeweiligen Becher enthaltenen Wasser ergaben. Nach Verdampfen des Acetons und einer Wartezeit von einer Stunde wurden etwa 50 viertägige Aedes-Larven in jeden der den Wirkstoff enthaltenden Becher eingesetzt. Die Ansätze wurden bei Raumteperatur behalten.

Zur Auswertung wurde nach 2, 4 und 24 Stunden sowie nach 8 Tagen auf Mortalität, d.h. den Prozentsatz der nicht mehr schwimmfähigen Larven, geprüft.

Verbindungen der vorstehenden Beispiele 1 und 2 zeigten gute Wirkung im obigem Test.

Beispiel 5
Wirkung auf Spodoptera littoralis (Eier):

Es wurde eine benetzbare, pulverförmige Formulierung enthaltend 25 Gew.-% des zu prüfenden Wirkstoffes hergestellt. Hieraus wurde durch Zugabe von Wasser eine benetzungsfähige Emulsions-Zubereitung mit einer Wirkstoffkonzentration von 800 ppm erhalten. Auf Filterpapier abgelegte, zwei Tage alte Eier von Spodoptera wurden in diese wässrige Wirkstoff-Zubereitung eingetaucht, herausgenommen und in Plastikschalen bei 28°C und 60% relativer Feuchtigkeit gehalten.

Die Auswertung erfolgte nach 4 Tagen durch Bestimmung der relativen Anzahl der aus den behandelten Eiern ausgeschlüpften Larven (prozentuale Schlupfrate).

Verbindungen der vorstehenden Beispiele 1 und 2 zeigten gute Wirkung im obigem Test.

Beispiel 6
Wirkung auf Leptinotarsa decemlineata (Larven):

Eingetopfte Kartoffelpflanzen im 4- bis 5-Blattstadium wurden in eine wässrige benetzungsfähige Emulsions-Zubereitung enthaltend 800 ppm des zu prüfenden Wirkstoffes eingetaucht. Nach dem Antrocknen wurden pro Pflanze 5 Leptinotarsa-Larven im 3.larvalen Stadium angesetzt. Plastikzylinder, deren obere Oeffnung mit Gaze

abgedeckt waren, wurden über die behandelten, mit Larven besiedelten Pflanzen gestülpt, um ein Abwandern der Tiere zu verhindern. Die Pflanzen wurden dann bei 28°C gehalten.

Die Auswertung erfolgte nach 3 Tagen unter Zugrundelegung der prozentualen Mortaliät der eingesetzten Larven (% Rückenlage).

Verbindungen gemäss den vorstehenden Beispielen 1 und 2 zeigten gute Wirkung im obigem Test.

Beispiel 7
Wirkung auf Spodoptera littoralis (Larven):

Eingetopfte Baumwollpflanzen im Keimblattstadium wurden in eine wässrige benetzungsfähige Emulsions-Zubereitung enthaltend 800 ppm des zu prüfenden Wirkstoffes eingetaucht. Nach dem Antrocknen wurden pro Pflanze 50 Spodoptera-Larven im 1. Stadium angesetzt. Plastikzylinder, deren obere Oeffnungen mit Gaze abgedeckt waren, wurden über die behandelten, mit Larven besiedelten Pflanzen gestülpt, um ein Abwandern der Tiere zu verhindern. Die Pflanzen wurden dann bei 28°C gehalten.

Die Auswertung erfolgte nach 3 Tagen unter Zugrundelegung der prozentualen Mortalität der eingesetzten Larven (% Rückenlage).

Verbindungen gemäss den vorstehenden Beispielen 1 und 2 zeigten gute Wirkung im obigem Test.

Beispiel 8

Wirkung auf Myzus persicae (Mischpopulation)

Keimlinge vom Pisum sativum wurden mit Myzus persicae infiziert, so dass eine Population in allen Entwicklungsstadien auf den keimenden Pflanzen vorlag. Jeweils 4 der infizierten Keimlinge wurden dann unter Wachstumsbedingungen in einem Becher fixiert und mit einer 0,05 Gew.-%igen Lösung des Wirkstoffes in einem Aceton-Wasser-Gemisch (1:1) besprüht, so dass die Oberfläche der infizierten Keimlinge mit einer gleichförmigen Schicht aus feinen Tröpfchen bedeckt war. Nach 2 und 7 Tagen wurden die Ergebnisse aufgezeichnet. Die behandelten Keimlinge wurden unter einem Stereomikroskop geprüft, um die prozentuale Abtötung der Myzus-Population zu bestimmen.

Verbindungen gemäss den vorstehenden Beispielen 1 und 2 zeigten gute Wirkung in obigem Test.

Beispiel 9

Ovizide Wirkung auf Heliothis virescens und Leptinotarsa decemlineata:

Entsprechende Mengenanteile einer benetzbaren pulverförmigen Formulierung, enthaltend 25 Gew.-% des zu prüfenden Wirkstoffes, wurden mit jeweils soviel Wasser vermischt, dass sich wässrige Emulsionen von ansteigender Wirkstoffkonzentration ergaben.

In diese wirkstoffhaltigen Emulsionen wurden eintägige Eigelege von Heliothis auf Cellophan bzw. Eigelege von Leptinotarsa auf Kartoffelblättern während drei Minuten eingetaucht und dann auf Rundfiltern abgenutscht. Die so behandelten Gelege wurden in Petrischalen ausgelegt und in der Dunkelheit aufbewahrt. Nach 6 bis 8 Tagen wurde die Schlupfrate im Vergleich zu unbehandelten Kontrollen festgestellt. Zur Auswertung wurde die zur 100%-igen Abtötung der Eier erforderliche minimale Wirkstoffkonzentration bestimmt.

Erfindungsgemässe Verbindungen gemäss den Beispielen 1 und 2 zeigten in diesem Test gute ovizide Wirkung gegen die geprüften Schädlinge.

Beispiel 10

Synergistische Insektizidwirkung auf resistente Stämme von musca domestica:

Es wurden acetonische Stammlösungen hergestellt, die eine erfindungsgemässe synergistisch wirksame Verbindung der Formel I sowie ein bekanntes Insektizid, wie z.B. 0,0-Diäthyl-0-(2-isopropyl-4-methyl-pyrimid-6-yl)-thionophosphorsäureester ("Diazinon"), Chrysanthemumsäure-[5-benzylfuryl-(3)-methylester] ("Bioresmethrin")oder 2-(1,3-Dioxolan-2-yl)-phenyl-methylcarbamat ("Dioxacarb"), im Gew.-Verhältnis 9,0 : 1,0, 7,5 : 2,5, 5,0 : 5,0, 2,5 : 7,5, und 1,0 : 9,0

enthielten. Zur Herstellung von Lösungen für sublethale Dosierungen wurden die entsprechenden acetonischen Stammlösungen so verdünnt, dass sich Anwendungslösungen ergaben, die pro 0,01 ml 75 bis 1500 Nanogramm der verwendeten Wirkstoffe (d.h. Synergist plus Insektizid) enthielten. Dann wurden dreitägige, weibliche, adulte, resistente oder multiresistente Fliegen durch Kälteschock inaktiviert und 0,01ml der jeweiligen acetonischen Anwendungslösung mittels einer Mikropipette auf die Oberseite des Abdomens eines jeden Versuchstieres appliziert. Die Dosierung der Wirkstoffe wurde sublethal gewählt, um den wirkungssteigernden Effekt des verwendeten Synergisten anhand der Erhöhung der prozentualen Mortalität der Versuchstiere aufzuzeigen.

Jeweils 20 der behandelten inaktivierten Fliegen wurden in eine Petrischale eingesetzt, die als Futter Zuckerwasser enthielt. Nach 24 Stunden wurde die prozentuale Mortalität bestimmt.

Verbindungen gemäss den vorstehenden Beispielen 1 und 2 zeigten gute synergistische Wirkung in obigem Test.

Patentansprüche

1. Verbindung der Formel I

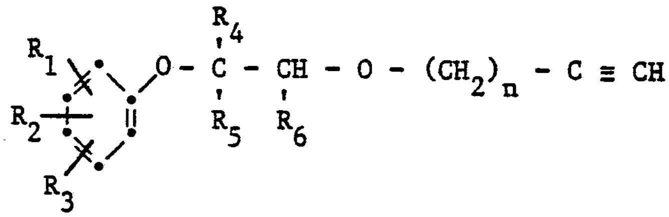 (I),

worin

$R_1$ Wasserstoff, $C_1$-$C_4$-Alkyl, Halogen, $C_1$-$C_4$-Alkoxy, $C_3$-$C_4$-Alkenyloxy, $C_3$-$C_5$-Alkinyloxy, Trifluormethyl, Nitro oder Cyano;

$R_2$ Wasserstoff, $C_1$-$C_4$-Alkyl, Halogen, $C_1$-$C_4$-Alkoxy, Trifluormethyl, Nitro oder zusammen mit $R_1$ Methylendioxy;

$R_3$ Wasserstoff, $C_1$-$C_4$-Alkyl oder Halogen;

$R_4$ Wasserstoff oder $C_1$-$C_4$-Alkyl;

$R_5$ Wasserstoff oder $C_1$-$C_4$-Alkyl;

$R_6$ Wasserstoff, $C_1$-$C_4$-Alkyl oder zusammen mit $R_5$ die $-(CH_2)_3-$ oder $-(CH_2)_4-$ Gruppe und

n eine der Zahlen 1, 2 oder 3 bedeuten.

2. Verbindung der Formel I gemäss Anspruch 1, dadurch gekennzeichnet, dass

$R_1$ Wasserstoff, $C_1$-$C_4$-Alkyl, Halogen, Methoxy, Aethoxy, Allyloxy, $C_3$-$C_4$-Alkinyloxy, Trifluormethyl oder Nitro;

$R_2$ Wasserstoff, Methyl, Halogen, Methoxy oder zusammen mit $R_1$ 3,4-Methylendioxy;

$R_3$ Wasserstoff, Methyl, Chlor oder Fluor;

$R_4$ Wasserstoff oder $C_1$-$C_4$-Alkyl;

$R_5$ Wasserstoff oder Methyl;

$R_6$ Wasserstoff, Methyl, Aethyl oder zusammen mit $R_5$ die $-(CH_2)_4-$ Gruppe und

n eine der Zahlen 1, 2 oder 3 bedeuten.

3. Verbindung der Formel I gemäss Anspruch 2, dadurch gekennzeichnet, dass

$R_1$ Wasserstoff, $C_1$-$C_4$-Alkyl, Chlor, Brom, Methoxy, Propargyloxy, Trifluormethyl oder Nitro;

$R_2$ und $R_3$ Wasserstoff, Methyl oder Chlor;

$R_4$ Wasserstoff oder Methyl;

$R_5$ Wasserstoff;

$R_6$ Wasserstoff, Methyl, Aethyl oder zusammen mit $R_5$ die $-(CH_2)_4-$ Gruppe und

n eine der Zahlen 1 oder 3 bedeuten.

4. Verbindung der Formel I gemäss Anspruch 1, 2 oder 3, dadurch gekennzeichnet, dass n die Zahl 1 bedeutet.

5. Verbindung der Formel I gemäss Anspruch 3, dadurch gekennzeichnet, dass

$R_1$ Wasserstoff oder in der 4-Stellung befindliches Chlor, Brom oder Propargyloxy;

$R_2$ und $R_3$ Wasserstoff oder Chlor;

$R_4$ und $R_5$ Wasserstoff;

$R_6$ Wasserstoff, Methyl oder Aethyl und

n die Zahl 1 bedeuten.

6. Verfahren zur Herstellung einer Verbindung gemäss den Ansprüchen 1 bis 5, dadurch gekennzeichnet, dass man eine Verbindung der Formel II

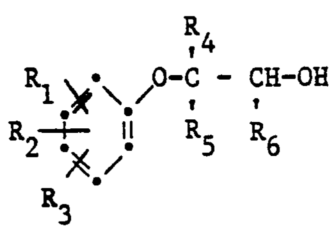

(II)

mit einer Verbindung der Formel III

$$Hal-(CH_2)_n-C\equiv CH \qquad (III)$$

umsetzt, wobei in den Formeln II und III die Reste $R_1$, $R_2$, $R_3$, $R_4$, $R_5$ und $R_6$ sowie n die in den Ansprüchen 1 bis 5 angegebenen Bedeutungen haben und Hal für ein Chlor- oder Bromatom steht.

7. Schädlingsbekämpfungsmittel, welches als aktive Komponente eine Verbindung gemäss den Ansprüchen 1 bis 5 zusammen mit geeigneten Trägern und/oder Zuschlagstoffen enthält.

8. Verwendung einer Verbindung gemäss den Ansprüchen 1 bis 5 zur Bekämpfung von Schädlingen, vorzugsweise von Insekten.

9. Verwendung einer Verbindung gemäss den Ansprüchen 1 bis 5 als Ovizide zur Bekämpfung pflanzenschädigender Insekten.

10. Mittel zur Bekämpfung von Schädlingen, welches als synergistisch wirksame Komponente eine Verbindung gemäss den Ansprüchen 1 bis 5 zusammen mit einem pestizid wirksamen Phosphorsäureester, Chlorkohlenwasserstoff, Carbamat oder Pyrethroid enthält.

Europäisches Patentamt

# EUROPÄISCHER RECHERCHENBERICHT

0012157

Nummer der Anmeldung

EP 79 10 3165

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | FR - A - 2 268 000 (CIBA-GEIGY)<br>* Anspruch 1 *<br>---- | 1 |

**KLASSIFIKATION DER ANMELDUNG (Int.Cl. 3)**

C 07 C 43/215
43/225
43/243
43/247
41/16
79/355
76/02
C 07 D 317/64
A 01 N 39/00
43/30//
C 07 C 43/23
41/03

**RECHERCHIERTE SACHGEBIETE (Int. Cl. 3)**

C 07 C 41/16
43/225
43/215
79/355

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 03-12-1979 | SAGATYS |